Europäisches Patentamt

European Patent Office

Office européen des brevets

(11) Publication number: **0 397 560**
A2

# EUROPEAN PATENT APPLICATION

(21) Application number: 90401228.3

(22) Date of filing: 09.05.90

(51) Int. Cl.⁵: **C12N 15/86, C12N 15/34, A61K 39/12, A01N 63/00**

(30) Priority: 08.05.89 US 348502
23.02.90 US 483735

(43) Date of publication of application:
14.11.90 Bulletin 90/46

(84) Designated Contracting States:
AT BE CH DE DK ES FR GB GR IT LI LU NL SE

(71) Applicant: Yuen, Kai-Chung Leonard
2070 De Maisonneuve W., Apt 56
Montreal, Quebec H3H 1K8(CA)

Applicant: Richardson, Christopher
3460 Peel Street., Apt 1711
Montreal, Quebec H3A 2M1(CA)

Applicant: Arif, Basil

27 Val St.
Sault-Ste-Marie, Ontario P6A 5B4(CA)

(72) Inventor: Yuen, Kai-Chung Leonard
2070 De Maisonneuve W., Apt 56
Montreal, Quebec H3H 1K8(CA)
Inventor: Richardson, Christopher
3460 Peel Street., Apt 1711
Montreal, Quebec H3A 2M1(CA)
Inventor: Arif, Basil
27 Val St.
Sault-Ste-Marie, Ontario P6A 5B4(CA)

(74) Representative: Lemoine, Michel et al
Cabinet Michel Lemoine et Bernasconi 13
Boulevard des Batignolles
F-75008 Paris(FR)

(54) Spheroidin DNA isolate and recombinant entomopoxvirus expression vectors.

(57) A DNA isolate consisting essentially of a DNA sequence encoding spheroidin and recombinant entomopoxvirus expression vectors capable of expressing a selected gene or portion thereof in tissue culture insect cells or insect larvae are described. These expression vectors consist of entomopoxvirus genomes comprising at least one selected gene or portion thereof. The selected gene or portion thereof is under the transcriptional control of an entomopox-virus promoter, preferably the spheroidin promoter. Also within the scope of the present invention is a method for producing viral transfer vectors, recombinant viral transfer vectors and recombinant viral expression vectors utilizing entomopoxvirus DNA segments. The recombinant entomopoxvirus expression vectors of the present invention are to be used for the production of proteins useful in medical, forestry and agricultural fields.

EP 0 397 560 A2

ATT TCA TAT TAT AAC TTA TAA TAC CAA TAT TTT ACT ACA ACT CTA ATA AAA ATA GAA TAA

TTT ATT TAT TAT AAA TAA GCA AAA ATA AAA AAA CAA ATA ATG AAT AAA TTA ATA CTA ATA
                                                MET Asn Lys Leu Ile Leu Ile

AGT CTT ATT GCG AGT TTA TAT CAA GTA GAA GTA GAC CCA CAC GCT TAT ATG ACA TTT CCT
Ser Leu Ile Ala Ser Leu Tyr Gln Val Glu Val Asp Ala His Gly Tyr MET Thr Phe Pro

ATA GCA AGA CAA AGA AGA TGT TCG GCG GCA GGC CGA AAT TGG TAT CCA GTA GGT GCA CGC
Ile Ala Arg Gln Arg Arg Cys Ser Ala Ala Gly Gly Asn Trp Tyr Pro Val Gly Gly Gly

GGT ATT CAA GAT CCG ATG TGT CGT GCC GCT TAT CAA AAT GTA TTC AAT AAA GTA TTA AAT
Gly Ile Gln Asp Pro MET Cys Arg Ala Ala Tyr Gln Asn Val Phe Asn Lys Val Leu Asn

TCT AAT GGA GGC GAC GTT ATA GAC GCG AGT GAA GCT CCT AAT TAT ATG TAT ACT CAG GAT
Ser Asn Gly Gly Asp Val Ile Asp Ala Ser Glu Ala Ala Asn Tyr MET Tyr Thr Gln Asp

AAC GAA TAT GCT GCT TTA GCT GGG CCA GAT TAT ACA AAT ATT TGT CAT ATC CAA CAA AGA
Asn Glu Tyr Ala Ala Leu Ala Gly Pro Asp Tyr Thr Asn Ile Cys His Ile Gln Gln Arg

GTA GTA CCT AGT TAT TTA TGT GCT GCT CGC GCT AGT GAT TGG TCT ATT AGA CCA TTC GGA
Val Val Pro Ser Tyr Leu Cys Ala Ala Gly Ala Ser Asp Trp Ser Ile Arg Pro Phe Gly

GAT AAA AGT GGT ATG GAT TTA CCG GCA AGT TGG ACA CCT ACT ATT ATA CAG TTG ACT GAT
Asp Lys Ser Gly MET Asp Leu Pro Gly Ser Trp Thr Pro Thr Ile Ile Gln Leu Ser Asp

AAT CAA CAA TCT AAT GTT GTA ATG GAA TTG GAA TTT TGT CCA ACA GCA GTA CAT GAT CCC
Asn Gln Gln Ser Asn Val Val MET Glu Leu Glu Phe Cys Pro Thr Ala Val His Asp Pro

AGT TAT TAC GAA GTA TAT ATA ACA AAT CCT AGT TTT AAT GTG TAT ACT GAT AAT GTG GTT
Ser Tyr Tyr Glu Val Tyr Ile Thr Asn Pro Ser Phe Asn Val Tyr Thr Asp Asn Val Val

TGG GCT AAC TTA GAT TTA ATA TAT AAT AAT ACA GTA ACT TTA AGA CCA AAA CTA CCT GAA
Trp Ala Asn Leu Asp Leu Ile Tyr Asn Asn Thr Val Thr Leu Arg Pro Lys Leu Pro Glu

TCT ACA TGT GCT GCT AAT TCT ATG GTT TAT ACA TTT GAA GTA TCG ATA CCT GTG ACA CCA
Ser Thr Cys Ala Ala Asn Ser MET Val Tyr Arg Phe Glu Val Ser Ile Pro Val Arg Pro

TCT CAA TTT GTA TTA TAT GTA AGA TGG CAA CBA ATC GAT CCT GTG GCA GAA GCA TTC TAC
Ser Gln Phe Val Leu Tyr Val Arg Trp Gln Arg Ile Asp Pro Val Gly Glu Gly Phe Tyr

AAC TGT GTC GAT ATG AAA TTT AAA TAT TCA GAA GGC CCC GAT GAA GAA GAT ATA ATT GAA
Asn Cys Val Asp MET Lys Phe Lys Tyr Ser Glu Gly Pro Asp Glu Glu Asp Ile Ile Glu

CCA GAA TAT GAA GTA GAT AAT CAG GCT GAA TGT TTT GCT TAT CGT TAT ACT AGT GGT AAT
Pro Glu Tyr Glu Val Asp Asn Gln Ala Glu Cys Phe Ala Tyr Arg Thr Asn Ser Gly Asn

GTG AAT GTT AAC CCA TTA CAA GAA AAT AAA TAT ATG GCA TAT GCC AAT AAA GCA ATT AGA
Val Asn Val Asn Pro Leu Gln Glu Asn Lys Tyr MET Ala Tyr Ala Asn Lys Ala Ile Arg

AAC ATA AAT ACC CAT TCT AAT GGT TGT AGT ACG AAT AGG AAT AAT AAA AAT AAT TAT AAT
Asn Ile Asn Thr His Ser Asn Gly Cys Ser Arg Asn Arg Asn Asn Lys Asn Asn Tyr Asn

AAA TAT TAT AGC AAA ACT TAT AAT TAT AAT CAA AAT ACA AAA TAA ATA TTT TAT TCA AAT
Lys Tyr Tyr Ser Lys Thr Tyr Asn Tyr Asn Gln Asn Arg Lys TER

TAT ATA AAT TGG AAT TTA ACC AAT TGA TAT TTA

FIGURE 3

2

## FIELD OF THE INVENTION

The present invention relates to a DNA isolate consisting essentially of a DNA sequence encoding spheroidin and to recombinant entomopoxvirus expression vectors capable of expressing selected genes or portion thereof in tissue culture insect cells or insect larvae.

## BACKGROUND OF THE INVENTION

Baculoviruses and entomopoxviruses are widely known insect viruses that have been isolated from a large number of insect species in widespread geographical locations. In recent years, both baculovirus and poxvirus vectors have achieved widespread acceptance for their ability to express proteins of agricultural and medical importance. For example, a baculovirus vector was used to express the first recombinant HIV envelope proteins to receive FDA approval for clinical evaluation as a candidate vaccine for AIDS. These insect DNA virus vectors are contributing to understanding the molecular biology of gene and protein function and regulation in both vertebrate and insect systems.

Poxvirus research, and more particularly the use of vaccinia virus, a prototypic member of the group of poxviruses, has led to eukaryotic cloning and expression vectors useful in a variety of biological and clinical applications. In 1982, Panicali and Paoletti reported in Proc. Natl. Acad. Sci., Vol. 1979, pp. 4927-4931 (August 1982), that endogenous subgenomic elements could be inserted into infectious progeny vaccinia virus via recombination in vivo. This ability to integrate vaccinia virus DNA sequences into infectious vaccinia virus progeny suggested the possibility for insertion of foreign genetic elements into infectious vaccinia virus via similar protocols. In order to test their assumption, Panicali and Paoletti inserted the herpes virus thymidine kinase (TK) gene into a number of vaccinia virus preparations and obtained pure cultures of recombinant vaccinia virus expressing the herpes virus gene.

Also in 1982, Hackett et al. in Proc. Natl. Acad. Sci., U.S.A. (December 1982) inserted foreign DNA into two non-essential regions of the vaccinia virus genome. Selection was achieved either by interrupting the endogenous TK gene of wild-type vaccinia virus or by adding the herpes virus TK gene to TK mutants.

In 1983, Smith and Moss in Gene 25 (1983), 21-28, reported that vaccinia virus appeared to have several advantages over other eukaryotic vectors. Most noteworthy was the fact that virus infectivity was not impaired by insertion and expression of foreign genes in contrast to defective SV40 and retrovirus vectors. The authors also mentioned that since vaccinia had served as a live smallpox vaccine, recombinants that expressed genes from organisms of current pathogenic importance might also be employed for medical or veterinary purposes. Moreover, even though the first candidate vaccine of this type expressed the hepatitis B virus surface antigen, the utility of the system could still be maximized by creating polyvalent recombinant vaccines containing several genes.

Furthermore, Smith and Moss recognized the fact that the size of the vaccinia virus genome suggested that the virus might have a large capacity for foreign DNA. They were able to insert into the vaccinia viral genome a DNA fragment having a length of 24.7 kb taken from bacteriophage lambda DNA. The recombinant virus thus obtained was stable and retained infectivity indistinguishable from wild-type virus.

Smith and Moss also acknowledged that although the upper limit of insert size was not known, the use of existing poxvirus deletion mutants as vectors would expand the capacity to at least 40 kb. At this time, vaccinia virus was the only described infectious eukaryotic expression vector capable of accommodating such large amounts of foreign DNA. This feature had considerable significance for the potential use of vaccinia recombinants as live vaccines for prevention of diseases in men and animals. Also, there was a possibility of producing polyvalent vaccines.

Thus, vaccinia virus has been successfully used as an expression vector through the insertion of foreign genes into a non-essential region of the viral genome via homologous recombination. However, some drawbacks have been associated with the use of this virus. First, vaccinia is a vertebrate pathogen that replicates at an optimum temperature of 37°C. Also, foreign antigens expressed in recombinant vaccinia viruses have to be rigorously purified from viral and host contaminants. Finally, the most difficult problem resides in the fact that vaccinia expression vectors are not capable of producing abundant foreign proteins because of the absence of known strong promoters.

Baculovirus vectors have also been used for the expression of foreign genes in insect cells. More specifically, transfer vectors for the expression of foreign genes under the control of the strong polyhedrin promoter of *Autographa Californica* nuclear polyhedrosis virus have been developed.

*Autographa Californica* nuclear polyhedrosis virus (AcNPV) is the prototype virus of the family *baculoviridae*. This virus has a wide host range and infects a large number of species of *Lepidopteran* insects. During AcNPV infection, two forms of viral progeny are produced.

The first form consists of extracellular virus particles (ECV) that are responsible for dissemination of the virus within the infected host by either endocytosis or fusion. The second form of viral progeny is an occluded virus particle (OV). These OV particles are embedded in proteinaceous viral occlusions. The major structural protein forming the occlusion matrix is a polyhedrin protein having a molecular weight of 29 000 Daltons.

These viral occlusions are an important part of the natural virus life cycle, providing the means for transmission of the virus from one host to another. They provide the virions a degree of protection against external environmental factors that would otherwise rapidly inactivate the extracellular virus particles. The occlusions dissolve in the alkaline environment of the insect gut, releasing the virus that invades and replicates in the cells of the midgut tissue.

AcNPV possesses several properties that make this virus ideally suited as an expression vector for cloned eukaryotic genes. Since occlusion of the virus is not absolutely essential for viral growth, the polyhedrin gene provides a non-essential region of the AcMNPV genome in which foreign DNA may be inserted. Also, the polyhedrin gene provides a very strong promoter which directs transcription late in infection after extracellular virus is produced and after host genes and most viral genes have been turned off. This gene also provides a genetic marker with which one may visually select recombinant viruses.

Using the properties of AcNPV, a wide variety of eukaryotic and prokaryotic genes have been expressed successfully with baculovirus vectors in insect cells. For example, Smith et al. reported in Mol. and Cell. Biology, Vol. 3, No. 12, (December 1983), pp. 2156-2165, the use of AcNPV as an expression vector to efficiently secrets biologically active human $\beta$ interferon in the media of infected cells.

Also, Pennock et al. in Molecular and Cell. Biology, Vol. 4, No. 3, (March 1984), pp. 399-406, demonstrated that AcNPV could be successfully employed as a recombinant DNA vector by stably propagating a 9.2 kb plasmid inserted at the polyhedrin gene site. They also constructed a plasmid containing the E. coli $\beta$-galactosidase gene, which could, as they suggested, facilitate the selection of recombinant viruses as blue plaques under semi-solid overlay medium containing the $\beta$-galactosidase indicator 5-bromo-4-chloro-3-indolyil-$\beta$-D-galactopyranosite (X-gal).

However, expression levels for different genes inserted into the same vector are often different and are thought to be related to the length and nature of the leader sequence preceding the foreign gene. In addition, different recombinant baculoviruses which contain a given foreign gene will often express that foreign gene at varying levels. Also, there may be a problem concerning the size of the gene placed in the expression vector, which may in some cases prevent the insertion and subsequent expression of large genes or multiple genes at the polyhedrin site.

Thus, the recombinant expression vectors constructed in recent years from both poxviruses and baculoviruses present drawbacks that can be summarized as follows. The poxvirus expression system often lacks a strong promoter and requires very rigorous conditions in order to obtain adequate expression. As for the baculovirus expression system, the size of the insertion site can create problems for the insertion of large or multiple genes.

Therefore the development of insect virus expression vectors necessitates the identification of a strong promoter and the capacity to incorporate and express large gene fragments.

## SUMMARY OF THE INVENTION

In accordance with the present invention, there is provided a DNA isolate consisting essentially of a DNA sequence encoding spheroidin. Preferably, the DNA sequence encodes the spheroidin gene of *Choristoneura biennis or Amsacta moorei*.

Also, there are provided recombinant entomopoxvirus expression vectors capable of expressing selected genes or portions thereof in tissue culture insect cells or insect larvae. These expression vectors consist of entomopoxvirus genomes comprising at least one selected gene or portion thereof, this selected gene or portion thereof being under the transcriptional control of an entomopoxvirus promoter or its own promoter.

Entomopoxviruses (EPVs) are members of the pox virus family. They have morphological similarities to poxviruses of vertebrates, and contain a large, linear, double-stranded DNA genome about 200 kilobase-pairs (kb) in size. Their natural hosts include insect larvae and when ingested, these virions are released from the occlusion bodies by alkaline environment of the insect midgut. To date, EPVs have been demonstrated to infect four different orders of insects, Lepidoptera, Coleoptera, Diptera, and Orthoptera. Together with the nuclear polyhedrosis viruses (NPVs), granulosis viruses (GVs), and cytoplasmic polyhedrosis viruses (CPVs), EPVs belong to the group of occluded insect viruses. Virions in this group are randomly embedded in proteinaceous occlusion bodies which consist of a major matrix protein named spheroidin.

With the present invention, some of the limitations encountered in prior art publications are overcome by providing an entomopoxvirus as well as a promoter within the entomopoxvirus genome to produce a viral expression vector in a host-vector system. More particularly, the present invention relates to the use of the entomopoxviruses *Choristoneura biennis* and *Amsacta moorei* and their associate spheroidin promoters to produce recombinant viral expression vectors capable of exceptionally high levels of expression of selected genes in tissue culture insect cells or insect larvae. Also, the very large genomic size of entomopoxviruses enables insertion of large foreign DNA fragments, thereby making the simultaneous incorporation of multiple genes possible. The present invention also relates to a method for synthesizing a selected polypeptide. This method comprises infecting susceptible host cells with a recombinant entomopoxvirus expression vector. The expression vector is an entomopoxvirus genome comprising at least one gene coding for the polypeptide or a portion thereof. The selection gene or portion thereof is under the transcriptional control of an entomopoxvirus promoter or its own promoter. The host cells are grown and the desired product is recovered.

Also within the scope of the present invention is a method for producing viral transfer vectors, recombinant viral transfer vectors and recombinant viral expression vectors utilizing an entomopoxvirus DNA segment. The resulting recombinant viral expression vectors will then be capable of expressing single selected genes or multiple genes in tissue culture insect cell or insect larvae.

The present invention also concerns the use of recombinant entomopoxviruses as pest control agents. Genes coding for substances that are deleterious to insect cells are to be inserted in the recombinant plasmids of the present invention in order to produce recombinant entomopoxviruses that will produce these substances and help to control the proliferation of undesirable pest insects. This will be obtained by spraying an insect infested area with a composition comprising the desired recombinant entomopoxvirus in admixture with an agriculturally and forestry acceptable carrier.

Also within the scope of the present invention is a method for the immunization of human or host animals through the use of genetically engineered entomopoxviruses vaccines. This method comprises immunizing a human or a host animal susceptible to entomopoxviruses by inducing an immunological response in the human or the animal. The immunological response is induced by inoculating the human or the animal with an entomopoxvirus synthetically modified by recombination to have, within a non-essential region of the entomopoxvirus genome, DNA not naturally occurring in the entomopoxvirus. This non-occurring DNA segment will be chosen among segments that code for an antigen that will induce the desired immunological response in the inoculated human or animal.

The present invention will be more readily illustrated by referring to the following description.

IN THE DRAWINGS

Figure 1A represents the electrophoresis gel of purified CbEPV occlusion bodies.

Figure 1B represents SA V8 protease digestions of CbEPV spheroidin monomer and dimer protein species.

Figure 2 represents the involvement of disulfide bonds in the formation and structure of CbEPV occlusion bodies.

Figure 3 represents the DNA sequence of the CbEPV spheroidin gene.

Figure 4 represents the hydropathy plot of CbEPV spheroidin.

Figure 5 represents the predicted secondary structure of CbEPV spheroidin.

Figure 6 represents a comparison between the 5′ flanking sequences of the CbEPV spheroidin gene and the vaccinia P4b gene.

DETAILED DESCRIPTION OF THE INVENTION

The present invention relates to a DNA isolate consisting essentially of a DNA sequence encoding spheroidin and to recombinant entomopoxvirus expression vectors capable of expressing selected genes or portion thereof in tissue culture insect cells or insect larvae. These expression vectors preferably comprise entomopoxvirus promoters, preferably the spheroidin promoter. The ability of the recombinant expression vector of the present invention to express proteins of agricultural, forestry and medical importance results from both the availability of a very strong promoter and the possibility to delete a large non-essential gene thereby creating available space for insertion of foreign genes.

Entomopoxviruses (EPV) include three possible subgenera represented by the type species *Melonlontha melonlontha* EPV (subgenus A), *Choristoneura biennis* EPV (CbEPV) and *Amsacta moorei* EPV (subgenus B) and *Chiromonus* EPV (subgenus C).

One of the distinguishing features of EPVs is their occlusion in a protein matrix at the end of the replication cycle in infected insect larvae or tissue culture cells. The protein matrix is composed primarily of a single polypeptide called spheroidin. Spheroidin is expressed in abundant quantities late in the infection cycle and is the product of a single gene.

When compared to polyhedrin, the equivalent of spheroidin in nuclear polyhedrosis viruses, no significant homology at the DNA or protein levels was found between spheroidin and polyhedrin. However, several structural and functional similarities exist between the two proteins. Hence, both proteins aggregate readily in solution. Also, both proteins are major components of their respective occlusion bodies and both are susceptible to alkaline conditions, resulting in the release of infectious virions.

However, as mentioned earlier, the availability of a strong spheroidin promoter as well as the large genomic size of entomopoxviruses allows insertion and expression of large foreign DNA fragments and renders possible the simultaneous incorporation of multiple genes.

The virions of EPVs are oval or brick-shaped with sizes ranging from 150-470 nm long and 165-300 nm wide. EPV virions have a folded mulberry-like outer membrane surrounding an electron-dense core containing the viral genome which is a linear double-stranded deoxyribonucleic acid (DNA). The DNA ranges in size from 225-333 kilobases.

Another distinguishing feature of EPVs is their biphasic replication cycle which produces two forms of the virus during infection of susceptible hosts. One form is destined to be occluded within the spheroidin matrix and is responsible for transmission of the virus from one host to another. Occlusion provides the virions with a degree of protection against harsh environmental factors. The second form of the virus is non-occluded and is responsible for dissemination of the virus within the infected hosts. Since occlusion is not required for virus growth, the spheroidin gene is considered non-essential and can be interrupted and used as a site for insertion of foreign genes.

Foreign genes that are important in pharmaceutical, industrial and pest-control applications can be genetically engineered into the EPV genome under the control of the spheroidin gene promoter. At the end of the viral replication cycle, large numbers of occlusion bodies containing mature virions are produced. For example, over $10^8$ occlusion bodies are present in one Spruce budworm larva infected with *Choristoneura biennis* EPV.

The spheroidin protein is the major component of the occlusion body matrix and it is coded for by one gene. The spheroidin gene is therefore under the control of a very strong promoter which is active late in infection that will allow high levels of expression of foreign genes engineered into the EPV genome.

Since the structural gene and promoter of spheroidin are the preferred DNA sequences to be used in the context of the present invention, it was necessary to purify and sequence the viral spheroidin gene.

The spheroidin protein was purified to near homogeneity by polyacrylamide gel electrophoresis. The N- terminus amino acid sequence of the gel-purified spheroidin protein was determined using techniques well known in the art. A DNA oligonucleotide corresponding to the amino acid sequence was chemically synthesized and used as a probe to locate the spheroidin gene.

The oligonucleotide was used in Southern blot analysis to hybridize to DNA fragments obtained by restriction digest of EPV genomic DNA. A 4.5 kb Xbal-Xbal *Choristoneura biennis* genomic DNA fragment was found to contain the spheroidin gene. Subcloning of the plasmid that contains the 4.5 kb insert allowed the identification of the spheroidin gene within a 2.3 kb EcoRI-Xbal fragment. This DNA fragment may be subjected to DNA sequencing to determine the coding and flanking regulatory sequences of the spheroidin gene.

Once the spheroidin gene has been sequenced, the spheroidin promoter can be utilized in expressing foreign genes by inserting foreign genes downstream of the promoter in the manner described thereafter. For example, the CbEPV spheroidin 5′ flanking sequence is believed to be a functional promoter in systems such as the vaccinia system. Indeed, preliminary data demonstrated that this 5′ spheroidin gene flanking sequence could function as a late vaccinia promoter when introduced into the vaccinia viral genome. This observation suggests that poxvirus late gene transcription mechanisms are highly conserved throughout evolution (from insect to mammalian hosts) of this class of viruses.

Generally speaking, the present invention will be put into practice by first cleaving entomopoxvirus DNA comprising an entomopoxvirus gene or a portion thereof including a promoter of this gene in order to obtain a DNA fragment that will contain the promoter. Preferably, DNA comprising the spheroidin gene and flanking DNA sequences of an appropriate entomopoxvirus such as *Choristoneura biennis* is isolated. The desired DNA is then cleaved by appropriate restriction enzymes. This will produce DNA fragments, some of which include the spheroidin promoter and at least one DNA sequence coding for the spheroidin promoter or a portion thereof.

Once the desired DNA fragment has been obtained, it is inserted into a suitable cloning vehicle which may be selected from the following plasmids: puc plasmids or related plasmids that contain selectable markers such as ampicillin or tetracycline resistant genes. The thus obtained transfer vector will then include a suitable cloning vehicle containing the spheroidin promoter as well as an available site for cloning a selected gene or portion thereof such that the selected gene is under the transcriptional control of the spheroidin promoter. The preferred transfer vector may also include DNA sequences coding for the spheroidin protein or a portion thereof. The entomopoxvirus sequences, which can range from approximately 500 to 5000 bp on either side of the insert, serve a vital function. They will be used to "direct" the foreign gene to the homologous sequences on the entomopox virus genome.

Once the transfer vector has been prepared, a recombinant transfer vector is then constructed by inserting the foreign gene of interest or a portion thereof into the available cloning site of the above-mentioned transfer vector. Any useful gene or genes may be cloned into the transfer vector of the present invention and coupled with an entomopoxvirus promoter sequence.

To obtain recombinant viruses, tissue culture insect cells are first infected with low doses of entomopoxviruses. After the virions have adsorbed and entered the host cells, recombinant transfer plasmids with foreign gene inserts will then be introduced into the infected cells via standard transfection procedures either by utilizing a calcium phosphate-precipitated preparation of plasmid DNA or by electroporation.

After uncoating of the virions in the cytoplasm of the infected cells and during viral DNA replication, the entomopoxvirus genome comes into close proximity of the recombinant plasmid DNA. The homologous entomopox sequences on the plasmid and on the viral genome will pair up by the process of in-vivo recombination. When this is accomplished, the virus genome will incorporate the foreign genes into its own DNA. Once this has occurred, replication of the recombined DNA molecule will continue, followed by maturation of the novel recombinant virus.

In order to facilitate screening of the recombinant viruses, the bacterial β-galactosidase gene will be inserted into the expression plasmid vector under the control of an entomopoxvirus promoter. Upon integration into the viral genome, β-galactosidase will give a blue coloration in the presence of its substrate X-gal. Thus, virus plaques that appear blue would represent recombinant viruses that contain the desired foreign genes. Recombinant viruses containing the desired proteins can then be purified and used to infect either tissue culture cells or insect larvae from which foreign proteins can be directly purified.

Apart from being a useful marker system for entomopoxvirus expression vectors, this marker was used in the construction of a baculovirus plasmid vector that was successfully used in expressing high levels of F and H proteins of the measles virus. The inclusion of the β-galactosidase gene in this recombinant plasmid allows easy identification of recombinant viruses since they appear as blue plaques in the presence of X-gal.

The recombinant entomopoxvirus expression vectors of the present invention can also be used in pest control application. This can be accomplished by including within the non-essential region of the entomopoxvirus genome one or more DNA sequences coding for substances that are deleterious to undesirable insects. Such substances can include for example *Bacillus thuringiensis* delta endotoxin, insect neurohormones or their analogs. The recombinant entomopoxviruses thus obtained will produce the deleterious substances and provide when combined with agriculturally acceptable carrier an efficient tool for controlling the proliferation of undesirable insects such as those of the *Choristoneura* species.

The recombinant entomopoxvirus expression vectors of the present invention can also be used to produce genetically engineered entomopoxviruses vaccines. This is accomplished by inserting within the non-essential region of the entomopoxvirus genome a DNA segment coding for an antigen capable of inducing the desired immunological response in the inoculated host. This leads to the development of a method for immunizing humans or host animals through inoculation of the host with live or attenuated entomopoxvirus vaccines synthetically modified by recombination to have, within a non-essential region of the entomopoxvirus genome, a DNA segment coding for an antigen that will induce the desired immunological response in the inoculated animal.

Alternatively, the recombinant entomopoxviruses of the present invention can be used to express desired foreign proteins which can be purified from tissue cultures cells or insect larvae. These proteins can then be used as vaccines (immunogens) to elicit immunological responses and protection against human and animal diseases.

The following examples are introduced to illustrate rather than limit the scope of the present invention.

Example 1

SEQUENCING OF THE CbEPV SPHEROIDIN PROTEIN.

### a) Purification of CbEPV virions and occlusion bodies.

CbEPV was propagated in *Choristoneura fumiferana* larvae. CbEPV occlusion bodies and virions were purified as previously described by Bilimoria and Arif in (1979) Virology 96, 596-603.

### b) Electrophoretic purification and protein sequencing of CbEPV spheroidin protein.

To purify spheroidin protein, CbEPV occlusion bodies were suspended in electrophoresis sample buffer (7 mM Tris-HCl, pH 7.4, 1% SDS, 10% glycerol, 1% $\beta$-mercaptoethanol ($\beta$-ME), and 0.05% bromphenol blue) and incubated at room temperature for 15 min. The protein samples were then boiled for 10 min and electrophoresed in a 10% sodium dodecyl sulfate (SDS)-polyacrylamide gel as described by Laemmli in (1970), Nature 227, 680-685. Electrophoresis was performed for 12 to 16 hr at 50 V.

The monomeric (50 kDa) form of spheroidin was located by Coomassie blue staining, excised, and electroeluted in an Elutrap apparatus (Schleicher & Schuell). Prior to electroelution, the gel slices were equilibrated in the elution buffer (50 mM Tris-Hcl, pH 8.8, and 0.1% SDS) for 30 min at room temperature. Electroelution of spheroidin was performed in the elution buffer at 60 V for 16 to 24 hr. The spheroidin protein was recovered between the membranes and precipitated with trichloroacetic acid (TCA), washed with acetone, and dried in a vacuum. The pellet was resuspended in 0.5% ammonium hydroxide and subjected to protein sequencing by Edman degradation using an Applied Biosystems Model 470A gas phase sequencer.

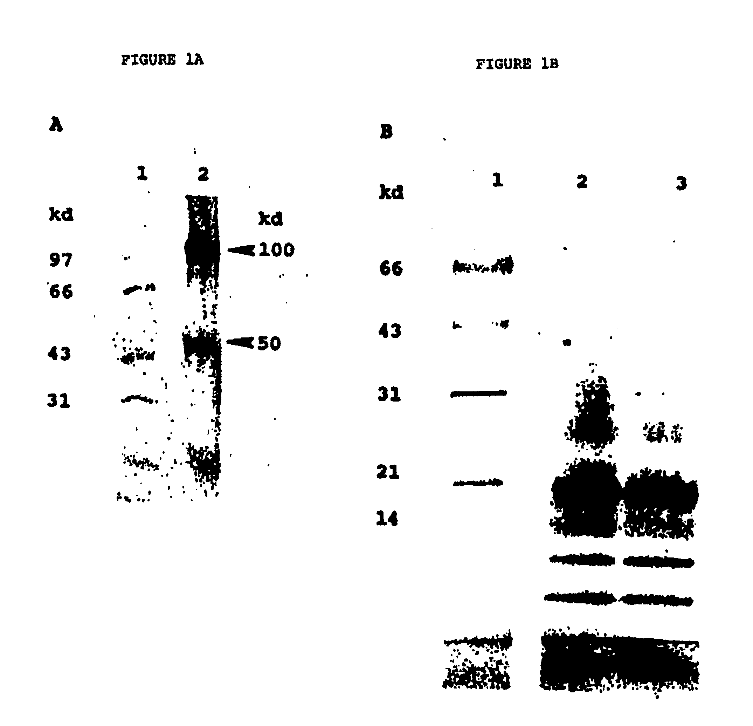

Two major spheroidin protein species with apparent molecular weights of 100 and 50 kDa could be detected as shown in Fig. 1A, lane 2 (lane 1 of Figure 1A shows protein molecular weight markers). A few minor proteins were also present. Most of them were found associated with purified CbEPV virions and thus presumed to be viral structural proteins. Previous reports suggested that EPV spheroidin was a protein with a molecular weight of 100 kDa as described by Bilimoria and Arif in (1979), Virology 96, 596-603. However, under the present reaction conditions, it was routinely noticed that while the ratio of the 100- and 50-kDa spheroidin protein species could be varied by simply changing the $\beta$-ME concentrations, no other change in the overall protein pattern was observed. To establish the relationship between these two protein species, they were subject to partial SA V8 protease peptide digestions. These digestions were performed as previously described by Cleveland et al. in (1977), J. Biol. Chem. 252, 1102-1106. The monomeric (50 kDa) and dimeric (100 kDa) forms of CbEPV spheroidin protein were gel purified, located by Coomassie blue staining, and excised. Gel slices containing these two protein species were introduced into the wells of a 20% SDS-polyacrylamide gel and overlayed with electrophoresis sample buffer containing 1 $\mu$g of SA V8 protease (Boehringer-Mannheim). Electrophoresis was stopped for 20 min when the bromphenol blue dye had travelled halfway into the stacking gel to allow V8 protease digestion to take place. Electrophoresis was resumed until the bromphenol blue dye reached the bottom of the gel. V8 protease-digested peptides were visualized by silver staining using the Bio-Rad silver staining kit. Results are shown in Fig. 1B.

The 100- (Fig. 1B, lane 2) and 50-kDa (Fig. 1B, lane 3) protein species clearly shared the same V8 protease peptide patterns (lane 1 shows protein molecular weight markers). Furthermore, when the same experiment was performed using trypsin protease, the partial tryptic peptide patterns of these two proteins were also identical. These observations, together with their respective molecular weights, suggested that the 100-kDa protein species is the dimeric form of the 50 kDa spheroidin protein.

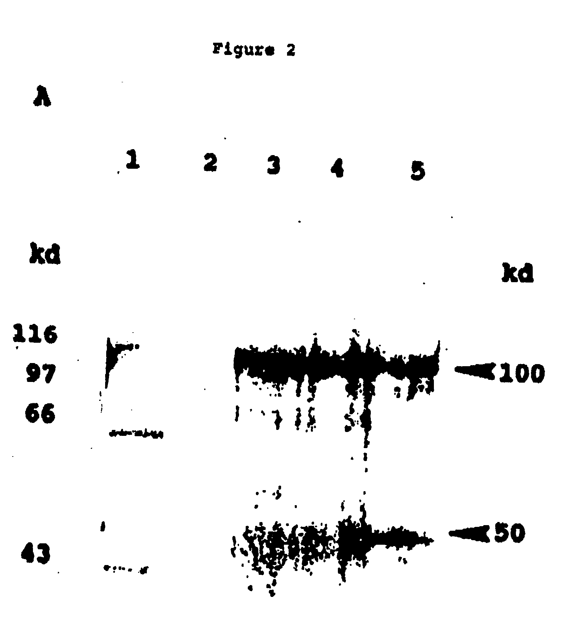

It was previously reported by McCarthy et al. in (1974), Virology 59, 59-69 that reducing agents were essential for disaggregating EPV occlusion body matrix, implicating the importance of intermolecular disulfide bonds on the matrix structure. To investigate the importance of disulfide bonds in the formation and structure of EPV occlusion bodies, CbEPV occlusion bodies were solubilized in electrophoresis sample buffer containing various amounts of $\beta$-ME, then subjected to 10% SDS-polyacrylamide gel electrophoresis and visualized by Coomassie blue staining. Results are shown in Fig. 2. In the absence of $\beta$-ME, all matrix proteins remained aggregated and stayed in the sample well as seen in Fig. 2, lane 2. Increasing $\beta$-ME concentrations (lane 3, 0.1% $\beta$-ME, lane 4: 0.2% $\beta$-ME and lane 5, 0.5% $\beta$-ME) resulted in gradual appearance of the 100-kDa spheroidin dimer and the 50-kDa monomer (lane 1 represents protein molecular weight markers). A shift from the 100-kDa dimer to the 50-kDa monomer was observed with higher concentrations of the reducing agent. This result confirmed the previous observation that reducing agents are required for solubilizing occlusion bodies; and it also demonstrated the importance of disulfide linkage in the dimerization and polymerization processes of the 50-kDa spheroidin monomeric protein. Other reducing agents such as dithiothreitol are also capable of solubilizing the occlusion body matrix.

Example 2

SEQUENCING OF THE CbEPV SPHEROIDIN GENE.

a) Purification of CbEPV DNA and construction of CbEPV genomic libraries

CbEPV genomic DNA was purified by suspending approximately $10^{10}$ CbEPV virions in a 2-ml volume containing 50 mM Tris-HCl, pH 7.8, 0.5% SDS, 6% sucrose (w/v), and 2 mg/ml proteinase K (Boehringer-Mannheim). The mixture was incubated at 65°C overnight. DNA was extracted three times with phenol-chloroform by gentle mixing of the two phases. Viral DNA was then ethanol precipitated and suspended in deionized water.

CbEPV genomic libraries were constructed by digesting purified viral DNA with HindIII, EcoRI or XbaI and cloned into pUC 19 vector digested with the respective enzymes.

b) Identification and DNA sequencing of the CbEPV spheroidin gene

To identify the spheroidin gene, the N-terminus of the 50-kDa spheroidin protein was sequenced. In addition to the electrophoretically purified 50-kDa protein species, N-terminus protein sequencing was also performed on purified CbEPV occlusion bodies that were solubilized in a buffer containing SDS, β-ME, and 8 M urea as previously described by Bilimoria and Arif in (1979), Virology 96, 596-603. Under this condition, the alkaline protease was reported to be inactivated. Direct protein sequencing of the spheroidin protein from purified occlusion bodies was possible because spheroidin is by far the most abundant protein species present. Indeed, N-terminus sequences of both the purified 50-kDa protein species and solubilized occlusion bodies were identical. A sequence of 30 amino acids was obtained and a degenerate oligonucleotide TAXATGACNTTXCCNAT (where X is either T or C and N is any one of the four nucleotides) corresponding to the peptide sequence Tyr.MET.Thr.Phe.Pro.Ile was synthesized in an Applied Biosystems Model 380A DNA synthesizer. CbEPV genomic DNA digested with XbaI was electrophoresed in an 0.8% agarose gel and transferred to nitrocellulose membrane (Schleicher & Schuell). The oligonucleotide was end-labelled with T4 polynucleotide kinase (Bethasda Research

Laboratory) and [γ -$^{32}$P]ATP (Amersham, 3000 Ci/mmol), and used as a probe in Southern blot analysis to locate the spheroidin gene on the XbaI-digested CbEPV genomic DNA. Hybridization was performed overnight at room temperature in 6X SSC (90 mM sodium citrate and 900 mM sodium chloride) and 100 μg/ml of yeast tRNA. Washings were performed in 2X SSC at room temperature.

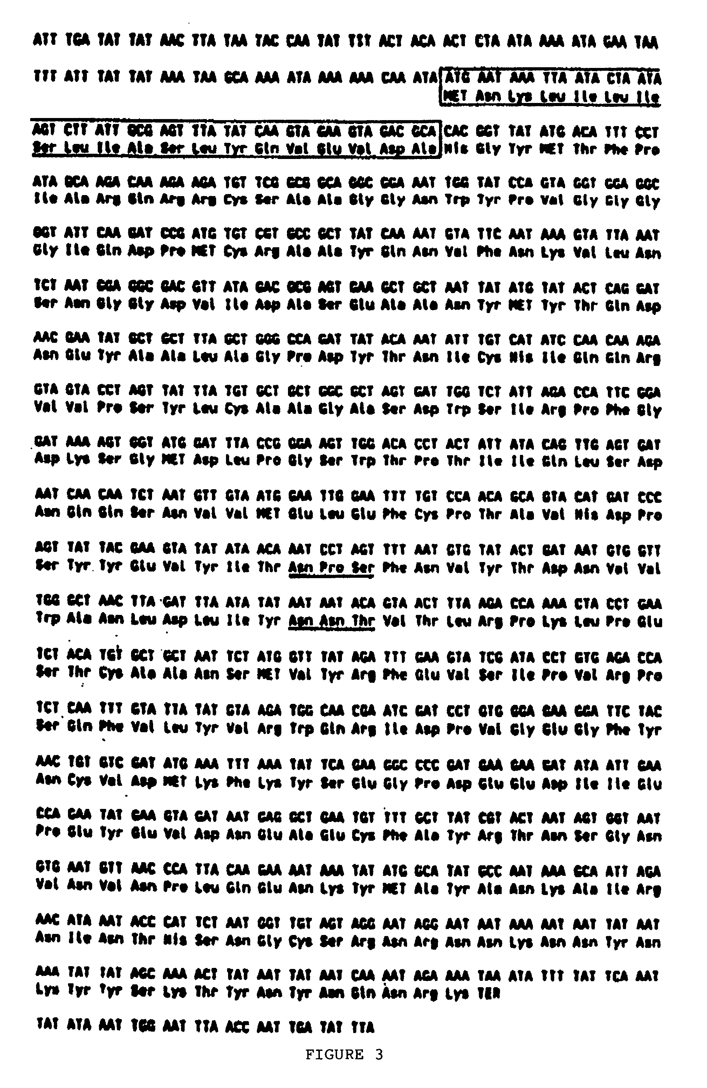

A 4.5-kb XbaI-XbaI genomic fragment was found to hybridize to the oligonucleotide. Subcloning of the plasmid that contains the 4.5-kb insert allowed identification of the spheroidin gene within a 2.3-kb EcoRI-XbaI fragment. DNA sequencing of this DNA fragment was performed on double-stranded plasmid DNA using synthetic oligonucleotide primers, [α-$^{32}$P]dATP (Amersham, 3000 Ci/mMol), and the T7 DNA polymerase sequencing kit from Pharmacia. Both DNA strands were sequenced to ensure the accuracy of the results. The DNA sequence of the open reading frame encoding the spheroidin gene is shown in Figure 3. In this figure, the CbEPV spheroidin gene and flanking sequences are shown. The signal peptide at the N-terminus is boxed and two potential N-glycosylation sites are underlined.

c) Characterizations of the CbEPV spheroidin gene

The CbEPV spheroidin gene is 1023 nucleotides in length and codes for a protein with a predicted molecular weight of 38.5 kDa. Mature spheroidin has an apparent molecular weight of 50 kDa, which is considerably larger than its predicted molecular weight. This discrepancy may be attributed to post-translational modifications. Since EPVs are cytoplasmic viruses like other poxviruses, splicing is likely not involved in mRNA formation, and thus, the genomic DNA sequence is expected to be identical to the mRNA sequence. Typical of poxvirus genes, the spheroidin gene is very AT rich (67%). It is acidic, with a predicted pH of 5.71. The protein sequence deduced from the DNA sequence is identical to the N-terminal protein sequence obtained by protein sequencing of the purified spheroidin protein except that the first 20 N-terminal amino acids are absent in the mature spheroidin protein molecules. Examination of these 20 amino acids revealed close resemblance to the consensus signal peptide sequence, with a highly hydrophobic region flanked by two more polar regions as shown by Heijne in (1986), Nucleic Acids Res. 14, 4683-4690. The presence of this signal peptide like sequence suggests the possibility that the CbEPV spheroidin is a secreted protein. However, due to the lack of an efficient tissue culture system for CbEPV at the present time. It is not possible to determine whether the CbEPV spheroidin protein is indeed secreted.

The predicted molecular weight of the mature spheroidin protein is 36 kDa (with 20 N-terminal amino acids removed), considerably smaller than its 50-kDa molecular weight in SDS-polyacrylamide gel. Such discrepancy can be due to post-translational modifications of the protein. In fact, two potential N-glycosylation sites are present on the spheroidin protein molecule (Fig. 3). Other common modifications such as phosphorylation, sulfation, and acetylation may also contribute to the observed increased molecular weight.

d) Computer analysis of the spheroidin protein

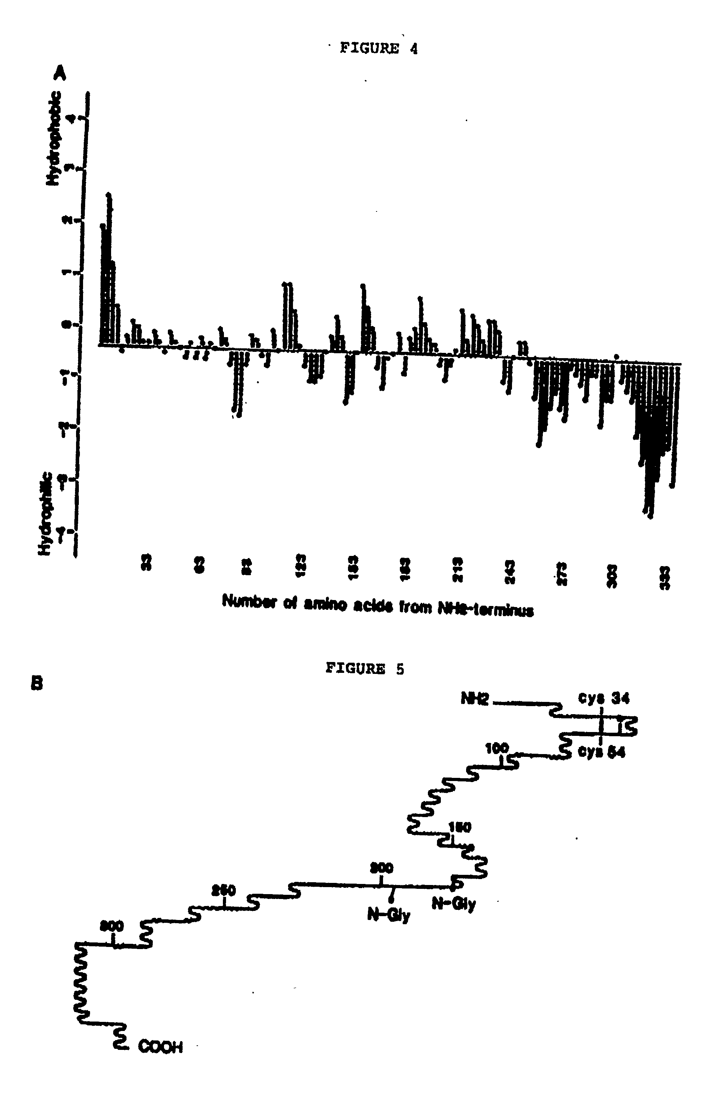

To further understand the spheroidin protein, computer analysis was performed. The hydropathy plot of spheroidin was obtained using the Pustell sequence analysis program. Results are shown in Figure 4. A prominent hydrophobic region resides in the signal peptide sequence region at the N-terminus of the protein. Conversely, the C-terminus of the spheroidin is mainly hydrophillic.

The Chou-Fasman prediction of the PlotStructure program was employed to predict the secondary structure of spheroidin. Results are shown in Figure 5. The hydrophobic N-terminus is likely to be membrane-associated, and, when cleaved by protease at the alanine (20) residue, forms the mature spheroidin molecule. Of the 9 cysteine residues in the spheroidin protein, 2 of them can potentially form intramolecular disulfide bond (residues 34 and 54), while the other 7 may participate in intermolecular disulfide bond formation. Figure 5 also shows the position of the two potential N-glycosylation sites. Helices are shown with sine waves $\beta$ sheets with sharp tooth waves and coils with dull saw tooth waves.

e) Homology between the 5′ flanking sequences of the CbEPV spheroidin gene and the vaccinia major core protein precursor gene P4b

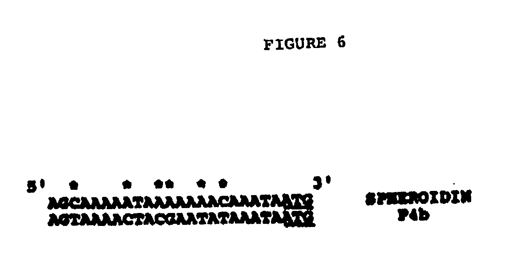

It was previously reported that vaccinia late genes possess a consensus TAAATG motif whereby the 3′ATG trinucleotide represents the translation initiation codon (Hanggi et al., (1986), Embo. J. 5, 1071-1076; Rosel et al., (1986), J. Virol. 60, 436-449). Transcription of vaccinia late genes were shown to initiate within this TAAATG hexanucleotide sequence. Homology between the 5′ flanking sequences of the CbEPV spheroidin gene and the vaccinia P4b is shown in Figure 6. Sequences (25 nucleotides) including the ATG initiation codon and upstream regions of the CbEPV spheroidin gene and the vaccinia P4b gene are shown. Mismatched nucleotides are denoted by asterisks and the ATG translation initiation codons are underlined. Examination of the CbEPV spheroidin gene revealed the presence of a similar sequence TAATG at the site of the ATG translation initiation codon. When comparing the 5′ flanking sequences of the CbEPV spheroidin gene with known vaccinia late genes, striking resemblance between the CbEPV spheroidin gene and the vaccinia major core protein precursor gene P4b was observed. Of the 25 nucleotides shown, 19 nucleotides are common to both genes, representing a 76% homology within this region (Fig. 6). Both sequences are very rich in deoxyadenosine residues (72% for spheroidin and 56% for P4b). Due to the substantial homology between the two genes in the region immediately upstream of the ATG initiation codon, and previous reports that short stretches of sequences (about 30 nucleotides) 5′ to the ATG codon in vaccinia late genes functioned sufficiently as vaccinia late promoters as described by Cochran et al. in (1985), J. Virol. 53, 30-37; Hanggi et al. in (1986), EMBO J. 5, 1071-1076, the CbEPV spheroidin 5′ flanking sequence is belived to be a functional promoter in the vaccinia system. Indeed, preliminary data demonstrated that this 5′ spheroidin gene flanking sequence could function as a late vaccinia promoter when introduced into the vaccinia viral genome.

Claims

1. A DNA isolate consisting essentially of a DNA sequence encoding spheroidin.

2. A DNA isolate according to claim 1, wherein the DNA sequence encoding spheroidin is an entomopoxvirus spheroidin DNA sequence.

3. A DNA isolate according to claim 2, wherein the entomopoxvirus is selected from *Choristoneura biennis* and *Amsacta moorei*.

4. A DNA isolate consisting essentially of the DNA sequence shown in Figure 3.

5. A recombinant entomopoxvirus expression vector capable of expressing a selected gene or portion thereof in tissue culture insect cells or insect larvae, said expression vector being an entomopoxvirus genome comprising at least one selected gene or portion thereof, said selected gene or portion thereof being under the transcriptional control of an entomopoxvirus promoter or its own promoter.

6. The recombinant entomopoxvirus expression vector of claim 5, wherein the entomopoxvirus promoter is a spheroidin promoter.

7. A recombinant transfer vector, capable of introducing a selected gene or portion thereof into an entomopoxvirus genome, said transfer vector comprising a cloning vehicle having a DNA sequence comprising an entomopoxvirus gene and at least one selected gene or portion thereof linked to said entomopoxvirus gene such that said gene or portion thereof is under the transcriptional control of a promoter of said entomopoxvirus gene or its own promoter.

8. The recombinant transfer vector of claim 7, wherein the entomopoxvirus promoter is a spheroidin promoter.

9. An entomopoxvirus transfer vector capable of being utilized as an intermediate vehicle for the genetic manipulation of an entomopoxvirus, said entomopoxvirus vector comprising an entomopoxvirus promoter gene and at least one available site for cloning a selected gene or portion thereof, said available cloning site being located such that the selected gene or portion thereof is under the transcriptional control of an entomopoxvirus promoter when inserted into said available cloning site.

10. The entomopoxvirus transfer vector of claim 9, wherein the entomopoxvirus promoter is a spheroidin promoter.

11. A method for producing a recombinant entomopoxvirus expression vector possessing the ability to express a selected gene or portion thereof in tissue culture insect cells or insect larvae, said method comprising:

a) cleaving entomopoxvirus DNA to produce a DNA fragment comprising an entomopoxvirus gene or portion thereof;

b) preparing a recombinant transfer vector by inserting said DNA fragment into a cloning vehicle and thereafter inserting at least one selected gene or portion thereof into the thus modified cloning vehicle such that said selected gene or portion thereof is under the transcriptional control of a promoter of said entomopoxvirus gene or its own promoter;

c) contacting said recombinant transfer vector with entomopoxvirus DNA through homologous recombination; and

d) isolating and recovering the desired recombinant entomopoxvirus expression vector.

12. The method of claim 11, wherein the selected gene or portion thereof is inserted into the cloning vehicle in place of at least a portion of the entomopoxvirus gene.

13. The method of claim 11, wherein the entomopoxvirus gene is a spheroidin gene or a portion thereof which includes the spheroidin promoter.

14. The method of claim 13, wherein the selected gene or portion thereof is inserted into the cloning vehicle in place of at least a portion of the DNA sequence coding for the synthesis of spheroidin.

15. The method of claim 11, wherein the entomopoxvirus is *Choristoneura biennis* or *Amsacta moorei*.

16. A method for reproducing a recombinant transfer vector having at least one selected gene or portion thereof introduced into an entomopoxvirus genome, said method comprising:

a) cleaving entomopoxvirus DNA to produce a DNA fragment comprising an entomopoxvirus gene or portion thereof;

b) inserting said DNA fragment into a cloning vehicle so as to produce an entomopoxvirus gene transfer vector and;

c) inserting at least one selected gene or portion thereof into said entomopoxvirus gene transfer vector, such that said gene or portion thereof is under the transcriptional control of said entomopoxvirus promoter or its own promoter.

17. The method of claim 16, wherein the selected gene or portion thereof is inserted into the cloning vehicle in place of at least a portion of the entomopoxvirus gene.

18. The method of claim 16, wherein the entomopoxvirus gene is a spheroidin gene or a portion thereof which includes a spheroidin promoter.

19. The method of claim 18, wherein the selected gene or portion thereof is inserted into the cloning vehicle in place of at least a portion of the DNA sequence coding for the synthesis of spheroidin.

20. The method of claim 16, wherein the baculovirus is *Choristoneura biennis* or *Amsacta moorei*.

21. A method for producing an entomopoxvirus transfer vector possessing the ability to be utilized as an intermediate vehicle for the genetic manipulation of entomopoxvirus DNA, said method comprising:

a) cleaving entomopoxvirus DNA to produce a DNA fragment containing at least a promoter;

b) inserting said DNA fragment into a cloning vehicle so as to produce a modified cloning vehicle having at least one available site for cloning a selected gene or portion thereof, said available cloning site being located such that said selected gene or portion thereof will be under the transcriptional control of said promoter when inserted into said available cloning site.

22. The method of claim 21, wherein the entomopoxvirus gene is a spheroidin gene or a portion thereof which includes a spheroidin promoter.

23. A method for synthesizing a selected polypeptide which comprises infecting susceptible host cells with a recombinant entomopoxvirus expression vector wherein said expression vector is an entomopoxvirus genome comprising at least one gene coding for said polypeptide or portion thereof, said selected gene or portion thereof being under the transcriptional control of an entomopoxvirus promoter or its own promoter, growing said host cells and recovering the desired product.

24. The method of claim 23, wherein the entomopoxvirus promoter is a spheroidin promoter.

25. A method for controlling the proliferation of pest insects in an area infested by said insects, said method comprising applying over said area a recombinant entomopoxvirus genetically modified to include within a non-essential region of its genome DNA coding for a substance deleterious to said insects, said recombinant entomopoxvirus being in admixture with an agriculturally acceptable carrier.

26. A method for the immunization of a human or a host animal comprising inoculating said human or host animal with live or attenuated entomopoxvirus vaccines synthetically modified by recombination to have, within a non-essential region of the entomopoxvirus genome, a DNA segment coding for an antigen that will induce the desired immunological response in the inoculated animal.

27. A method for the immunization of a human or a host animal comprising inoculating said human or host animal with a purfied immunogen produced by tissue culture cells or insect larvae which have been previously infected with a recombinant entomopoxvirus expression vector comprising a gene coding for said immunogen, said gene being under the transcriptional control of an entomopoxvirus promoter.

FIGURE 1A

FIGURE 1B

Figure 2

EP 0 397 560 A2

ATT TGA TAT TAT AAC TTA TAA TAC CAA TAT TTT ACT ACA ACT CTA ATA AAA ATA GAA TAA

TTT ATT TAT TAT AAA TAA GCA AAA ATA AAA AAA CAA ATA ATG AAT AAA TTA ATA CTA ATA
                                                   MET Asn Lys Leu Ile Leu Ile

AGT CTT ATT GCG AGT TTA TAT CAA GTA GAA GTA GAC GCA CAC GGT TAT ATG ACA TTT CCT
Ser Leu Ile Ala Ser Leu Tyr Gln Val Glu Val Asp Ala His Gly Tyr MET Thr Phe Pro

ATA GCA AGA CAA AGA AGA TGT TCG GCG GCA GGC GGA AAT TGG TAT CCA GTA GGT GGA GGC
Ile Ala Arg Gln Arg Arg Cys Ser Ala Ala Gly Gly Asn Trp Tyr Pro Val Gly Gly Gly

GGT ATT CAA GAT CCG ATG TGT CGT GCC GCT TAT CAA AAT GTA TTC AAT AAA GTA TTA AAT
Gly Ile Gln Asp Pro MET Cys Arg Ala Ala Tyr Gln Asn Val Phe Asn Lys Val Leu Asn

TCT AAT GGA GGC GAC GTT ATA GAC GCG AGT GAA GCT GCT AAT TAT ATG TAT ACT CAG GAT
Ser Asn Gly Gly Asp Val Ile Asp Ala Ser Glu Ala Ala Asn Tyr MET Tyr Thr Gln Asp

AAC GAA TAT GCT GCT TTA GCT GGG CCA GAT TAT ACA AAT ATT TGT CAT ATC CAA CAA AGA
Asn Glu Tyr Ala Ala Leu Ala Gly Pro Asp Tyr Thr Asn Ile Cys His Ile Gln Gln Arg

GTA GTA CCT AGT TAT TTA TGT GCT GCT GGC GCT AGT GAT TGG TCT ATT AGA CCA TTC GGA
Val Val Pro Ser Tyr Leu Cys Ala Ala Gly Ala Ser Asp Trp Ser Ile Arg Pro Phe Gly

GAT AAA AGT GGT ATG GAT TTA CCG GGA AGT TGG ACA CCT ACT ATT ATA CAG TTG AGT GAT
Asp Lys Ser Gly MET Asp Leu Pro Gly Ser Trp Thr Pro Thr Ile Ile Gln Leu Ser Asp

AAT CAA CAA TCT AAT GTT GTA ATG GAA TTG GAA TTT TGT CCA ACA GCA GTA CAT GAT CCC
Asn Gln Gln Ser Asn Val Val MET Glu Leu Glu Phe Cys Pro Thr Ala Val His Asp Pro

AGT TAT TAC GAA GTA TAT ATA ACA AAT CCT AGT TTT AAT GTG TAT ACT GAT AAT GTG GTT
Ser Tyr Tyr Glu Val Tyr Ile Thr Asn Pro Ser Phe Asn Val Tyr Thr Asp Asn Val Val

TGG GCT AAC TTA GAT TTA ATA TAT AAT AAT ACA GTA ACT TTA AGA CCA AAA CTA CCT GAA
Trp Ala Asn Leu Asp Leu Ile Tyr Asn Asn Thr Val Thr Leu Arg Pro Lys Leu Pro Glu

TCT ACA TGT GCT GCT AAT TCT ATG GTT TAT AGA TTT GAA GTA TCG ATA CCT GTG AGA CCA
Ser Thr Cys Ala Ala Asn Ser MET Val Tyr Arg Phe Glu Val Ser Ile Pro Val Arg Pro

TCT CAA TTT GTA TTA TAT GTA AGA TGG CAA CGA ATC GAT CCT GTG GGA GAA GGA TTC TAC
Ser Gln Phe Val Leu Tyr Val Arg Trp Gln Arg Ile Asp Pro Val Gly Glu Gly Phe Tyr

AAC TGT GTC GAT ATG AAA TTT AAA TAT TCA GAA GGC CCC GAT GAA GAA GAT ATA ATT GAA
Asn Cys Val Asp MET Lys Phe Lys Tyr Ser Glu Gly Pro Asp Glu Glu Asp Ile Ile Glu

CCA GAA TAT GAA GTA GAT AAT GAG GCT GAA TGT TTT GCT TAT CGT ACT AAT AGT GGT AAT
Pro Glu Tyr Glu Val Asp Asn Glu Ala Glu Cys Phe Ala Tyr Arg Thr Asn Ser Gly Asn

GTG AAT GTT AAC CCA TTA CAA GAA AAT AAA TAT ATG GCA TAT GCC AAT AAA GCA ATT AGA
Val Asn Val Asn Pro Leu Gln Glu Asn Lys Tyr MET Ala Tyr Ala Asn Lys Ala Ile Arg

AAC ATA AAT ACC CAT TCT AAT GGT TGT AGT AGG AAT AGG AAT AAT AAA AAT AAT TAT AAT
Asn Ile Asn Thr His Ser Asn Gly Cys Ser Arg Asn Arg Asn Asn Lys Asn Asn Tyr Asn

AAA TAT TAT AGC AAA ACT TAT AAT TAT AAT CAA AAT AGA AAA TAA ATA TTT TAT TCA AAT
Lys Tyr Tyr Ser Lys Thr Tyr Asn Tyr Asn Gln Asn Arg Lys TER

TAT ATA AAT TGG AAT TTA ACC AAT TGA TAT TTA

FIGURE 3

FIGURE 4

FIGURE 5

FIGURE 6

5'  •    •  ••  •  •                        3'

AGCAAAAATAAAAAAACAAATAATG          SPHEROIDIN

AGTAAAACTACGAATATAAATAATG              P4b